# EUROPEAN PATENT SPECIFICATION

(11) **EP 1 317 495 B1**
(45) Date of publication and mention of the grant of the patent: **01.03.2006**
(21) Application number: 01949451.7
(22) Date of filing: 29.06.2001
(51) Int. Cl.: C08G 18/32

(54) **ISOCYANATE COMPOSITIONS AND THEIR USE IN THE PREPARATION OF EXPANDED POLYURETHANE MATERIALS WITH ENDOWED FLAME-RESISTANCE**
ISOCYANAT-ZUSAMMENSETZUNGEN UND IHRE VERWENDUNG IN DER HERSTELLUNG VON GEBLÄHTEN POLYURETHANMATERIALIEN AUSGESTATTET MIT FLAMMRESISTENZ
COMPOSITIONS D'ISOCYANATE ET UTILISATION ASSOCIEE DANS LA PREPARATION DE MATERIAUX EN POLYURETHANNE EXPANSE A RESISTANCE A LA FLAMME

(30) Priority: 29.06.2000 IT MI001456
(43) Date of publication of application: 11.06.2003
(73) Proprietor: DOW GLOBAL TECHNOLOGIES INC., Midland, Michigan 48674 (US)
(72) Inventor: PETRONE, Antonio, Spinea (IT); GREGO, Sandra, Salzano (IT); OKON SAM, Felix, Ponto San Nicolo (IT)
(74) Representative: Raynor, John
(86) International application number: PCT/EP2001/007477
(87) International publication number: WO 2002/000751

(56) References cited:
- US-A- 3 943 158
- US-A- 4 340 712
- US-A- 5 166 185
- US-A- 5 288 768

## Description

The present invention relates to an isocyanic composition and its use in the preparation of flexible expanded polyurethane material with endowed flame-resistance.

The term "flexible expanded polyurethane materials with endowed flame-resistance", as used herein refers to block and moutded (hot and cold) polyurethane expanded products and foams capable of providing flame-resistance performance which passes, for example, the CSE RF4 test, even up to the 1.1M classification, without the use of any flame retardant of the halogenated or phospho-halogenated type, and without the use of auxiliary additives such as melamine and its derivatives.

Both block and moulded flexible polyurethane foams are used in furniture and in the car industry. It is recognised that flame-resistance performance is especially important in these fields.

Flame retardancy may be obtained by using flame retardant additives which are pre-dispersed in the polyurethane reagents. For example GB-A-2,163,762 describes a process for the preparation of flexible polyurethane foams according to which an isocyanic component is reacted with a polyol component in the presence of an expanding agent and a flame-retardant additive. The flame-resistant polyurethane foams may be obtained using melamine as a flame-retardant additive and a modified polyol.

Russian patent SU 729,207 describes a process for preparing flame-resistant polyurethane foams which comprises the use of a flame retardant additive, selected from bis (chloromethyl)-phosphonates, pre-dispersed in one of the two polyurethane reagents.

U.S.-A-4,425,447 describes the use of tribromocumene as a flame-retardant agent for polyurethane foams.

The presence of a flame retardant agent or additive in a polyurethane foam, whilst providing satisfactory flame-resistance properties may introduce disadvantages, for example, poorer physico-mechanical properties of the foam itself or the discharge of toxic fumes in the case of combustion.

In EP-A-884,340 the problem of the presence of flame retardant additives or agents is said to be overcome by using an isocyanic component selected so as to give the end polyurethane foam flame-resistance characteristics without having to resort to the use of particular additives. Said isocyanic component consists of a mixture which comprises:
- 20-30% by weight of toluene diisocyanate (TDI);
- 30-50% by weight of TDI oligomers with an isocyanic functionality ranging from 3 to 4;
- 30-40% by weight of diphenylmethane diisocyanate (MDI) with a content of 2,4' isomers higher than 40% by weight

To reduce or avoid drawbacks associated with the use of flame retardant agents in the art and to provide an alternative to the solution proposed in EP-A-884,340, the Applicant has found that polyurethane expanded products or foams with a high flame-resistance may be secured without necessarily having to resort to particular flame retardant agents or additives, by using a new isocyanic composition suitably modified to be able to be used in a combination with a polyol component to produce the polyurethane.

The invention provides, in a first aspect, the use of an isocyanic component which is obtainable from the reaction of at least one organic isocyanate which is at least bifunctional with a secondary amine having general formula (I): 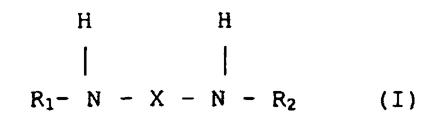
wherein R₁ and R₂ are independently, the same or different and represent a C₁₋C₈ (iso) alkyl radical and X represents a 4,4'-methylenediphenylene radical and wherein the molar ratio between the NCO groups of the said isocyanate and functional amine groups in the amine of formula (I) in from 2 to 10 in the preparation of a flexible expanded polyurethane material endowed with flame resistance.

Preferably, the isocyanic component has isocyanic functionality from 15 to 40%.

A preferred isocyanic component for use according to the present invention suitably has a viscosity at 25°C from 40 to 10,000 mPa.sec.

Secondary amines according to formula (I) are known per se and examples are described in for example US-A-5,166,185. Secondary amines of formula (I) may be prepared with conventional methods such as those described, for example, in J. March, "Advanced Organic Chemistry", second Edition, McGraw-Hill Kogakusha, 1977. A product having a linking group joining two secondary amine groups is also available on the market under the trade name of UNILINK 4200 of UOP.

The invention further provides an article comprising an expanded flexible polyurethane material of the present invention.

According to the present invention, any organic isocyanate which is at least bifunctional, may be used in the preparation of the isocyanic component of the present invention. Preferably, diisocyanates having a low or medium molecular weight having general formula (II), are used:

OCN - R - NCO (II)

wherein R represents a C₁-C₁₂ (iso) alkyl, a C₅-C₁₅ cycloalkyl or a C₆-C₁₈ aromatic radical which is optionally substituted with a C₁-C₄ alkyl radical. Examples of these products are hexamethylene diisocyanate, meta phenylene diisocyanate, para-phenylene diisocyanate, 2,4-toluenediisocyanate (TDI), 2,6-toluenediisocyanate, 4,4'-diphenylmethane-diisocyanate (MDI), 2,4' diphenylmethane-diisocyanate, 4,4'-dicyclohexylmethane diisocyanate, and 1-isocyanate-3-isocyanatemethyl-3,3,5-trimethylcyclohexane. Mixtures of these products may be employed if desired, for example 2,4-toluenediisocyanate and 2,6-toluenediisocyanate, 4,4'-diphenylmethane-diisocyanate and 2,4'-diphenylmethane-diisocyanate.

Polyisocyanates with a high molecular weight may also be employed, for example those compounds obtained by the phosgenation of aniline-formaldehyde condensates. The number of condensation units in such compounds which are suitable for use in the present invention may vary. These products are polymethylenepolyphenyl polyisocyanates having general formula (III): 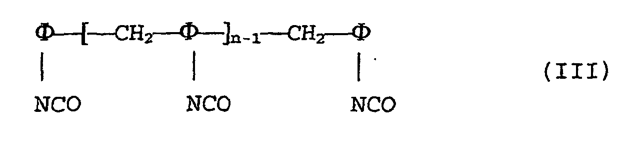
wherein Φ represents a phenyl group and n an integer greater than or equal to 1.

Preferred polyisocyanates with a medium or high molecular weight according to the present invention include polymethylenepolyphenyl polyisocyanates with an average functionality ranging from 2.6 to 2.8. These products are available on the market under various trade names such as "TEDIMON 31" (Enictuem S.p.A.), "SUPRASEC DNR" (ICI) or DESMODUR 44 V20 (Bayer).

Further examples of suitable polyisocyanates include isocyanic prepolymers obtained by reacting an excess in equivalents of one or more isocyanates having general formula (II) or (III) with at least one polyol polyether and/or polyester, optionally containing mixed ether or ester groups and/or groups of an aminic nature, having a functionality ranging from 2 to 8 and a molecular weight ranging from about 50 to 2000, of which a more detailed description follows.

A further object of the present invention relates to a process for the preparation of a flexible expanded polyurethane material endowed with flame resistance, which comprises reacting:
a) an isocyanic component obtainable, and preferably obtained from the reaction of at least one organic isocyanate which is at least bifunctional, as previously described herein, with a secondary amine having the general formula (I): 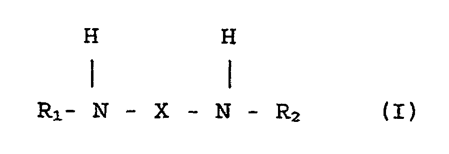
   wherein R1 and R₂, independently are the same or different and, represent a C₁-C₈ (iso)alkyl radical and X represents a 4,4'-methylenediphenylene radical and wherein the molar ratio between the NCO groups and functional amine groups of formula (I) in from 2 to 10; with
b) a polyol component which comprises at least one polyol with a hydroxyl functionality from 2 to 8 and a molecular weight from 50 to 2,000.

The polyol used in the preparation of the flexible expanded products according to the process object of the present invention, is suitably selected from polyol polyethers, polyol polyethers containing an ester group, polyol polyethers containing an amine group, polyol polyesters, polyol polyesters containing an ester group, and polyol polyesters containing an amine group. Preferred polyols include polyol polyethers obtained by the condensation of C₂-C₆ olefinic oxides on compounds having at least two active hydrogen atoms referred to herein as "starters". Preferred olefinic oxides are ethylene oxide, propylene oxide and mixtures thereof.

Suitably the condensation takes place on starters such as glycols, triols, tetrols, amines, alkanolamines, polyamines and mixtures thereof.

Representative examples of polyol polyethers which can be used according to the present invention include those based on ethylene oxide and/or propylene oxide and in which the starter is a glycol such as dipropylene glycol; a triol such as glycerin or trimethylolpropane; a tetrol such as pentaerythritol; a diamine such as ethylenediamine, an aromatic amine such as orthotoluenediamine, an alkanolamine such as triethanolamine, or a polyfunctional hydroxy alkane such as xylitot, arabitol, sorbitol, mannitol.

These polyols may be used as such in the process of the invention or they may contain in dispersion or partially grafted to the polyol chains, solid partides, preferably polymeric, with dimensions of less than 20 micrometers. Polymers suitable for this purpose include polyacrylonitrile, polystyrene and polyvinylchloride and mixtures thereof or copolymers, or urea-based polymers. These solid particles may be prepared by polymerization in situ in the polyol or they may be prepared separately and subsequently added to the polyol.

The polyol composition suitably also comprises further additives commonly used in the preparation of expanded polyurethanes such as amine catalysts, for example, triethylenediamine, and/or metallic catalysts such as stannous octoate, cell-regulators, thermo-oxidation stabilizers, pigments, and the like. Details on the polymerization of polyurethanes are provided in the text "Saunders & Frisch - Polyurethanes, Chemistry and Technology", Interscience, New York, 1964.

An expanding agent is suitably employed in the production of an expanded polyurethane material according to the process of the present invention. Suitably, the expanding agent comprises water, which may be used alone or in combination with a secondary expanding agent. In the preparation of expanded polyurethanes, water causes the formation of ureic bonds associated with the development of carbon dioxide which produces the expansion/swelling process of the polyurethane resin, obtaining flexible expanded products. Quantities of water from 1 to 10, preferably from 2 to 7 and even from 3 to 6 parts by weight with respect to 100 parts of polyol component are suitable.

Where water is employed as the expanding agent, carbon dioxide developed in situ by the chemical reaction between water and the NCO groups of the polyisocyanate is preferably used as primary agent for the expansion of the polyurethane resin. Other methods for introducing the primary expanding agent into the polymerization mass may be employed. Thus gases other than carbon dioxide and other techniques may be used, for example, the bubbling of air, liquid CO₂, nitrogen, fluorocarbon or another inert gas, into the reaction mass by external injection.

In the preparation of expanded polyurethane materials with a reduced density, for example having a density equal to or lower than 25 kg/m³, the expanding function of water alone may not be sufficient to reach such a density value without certain problems for example scorching, due to the exothermic reaction between water and the isocyanic groups. For this reason, the expanding action of water can be supported by expanding agents of a physical nature, selected from hydrofluoro alkanes, for example, 1,1,1,2-tetrafluoroethane (HFC-134a) liquid CO₂, hydrocarbons such as n-pentane, i-pentane, cyctopentane, dimethylcarbonate and mixtures thereof.

The flexible expanded polyurethane materials obtained according to the process of the present invention, suitably have a density from 20 to 200 kg/m³, preferably from 30 to 120 kg/m³, and an aerodynamic lift (according to the regulation ISO 2439) higher than 40 N, preferably from 80 to 400 N. Furthermore the materials suitably have no thermo-oxidative degradation phenomena of the scorching type and have excellent mechanical properties such as, ultimate elongation, elastic failure, compression strength and air permeability. Foams of the present invention may advantageously be used in the furniture, household furnishing and transport and car industries which typically require materials having these properties. Additionally, when subjected to flame-resistance tests, foams of the present invention desirably pass the CSE RF4 test up to the classification 1.IM.

The invention is illustrated by the following non-limiting examples.

In the examples, the quantities of the various components of the formulations are expressed as parts by weight, unless otherwise specified.

### EXAMPLE 1

43.8 kg of Tedimon 307 (mixture of 4,4' and 2,4' MDI in a ratio of 50/50) were charged into a reactor filled with nitrogen, equipped with stirring and a cooling system, and subsequently heated to 40°C. 6.2 kg of UNILINK 4200 of UOP was slowly added under vigorous stirring to this product. The reaction was exothermic and the temperature of the reaction mass was maintained at 70°C, the amine being fed at a rate of 0.28 kg/min.

The reaction mass was maintained at this temperature for 1.5 hours. The product (A) was subsequently discharged and analyzed, providing the following characteristics:
NCO % = 26.1;
Viscosity 25°C = 240 mPa.sec;
Aspect: yellow liquid;
Crystallization point = 0°C.

### EXAMPLE 2

The same procedure was adopted as described in Example 1 except for the use of 43.8 kg of Tedimon 306 (mixture of 4,4' and 2,4' MDI in a ratio of 80/20) which was preheated to 50°C.

The product (B) was discharged and analyzed, providing the following characteristics:
NCO % = 25.9;
Viscosity 25°C = 210 mPa.sec;
Aspect: yellow/green liquid;
Crystallization point = 20°C.

### EXAMPLE 3

42 kg of Tedimon 80 (mixture of 2,4 and 2,6 MDI in a ratio of 80/20) was charged into a reactor filled with nitrogen, equipped with stirring and a cooling system, and was subsequently heated to 40°C. 8 kg of UNILINK 4200 was slowly added to this product under vigorous stirring. The reaction was exothermic and the temperature of the reaction mass was maintained at 70°C, the feeding rate of the amine being controlled.

The reaction mass was maintained at this temperature for 1.5 hours. The product (C) was subsequently discharged and analyzed, providing the following characteristics:
NCO % = 36;
Viscosity 25°C = 60 mPa.sec;
Aspect: yellow/green liquid;
Crystallization point = 15°C.

### EXAMPLE 4

20.8 kg of Tedimon 306 and 20.8 kg of Tedimon 307 preheated to 50°C was charged into a reactor filled with nitrogen, equipped with stirring and a cooling system. The products were reacted with 2.5 kg of TERCAROL 241 (oxyethylene/oxypropylene triol with a molecular weight of 4,000) for at least 30 minutes at 70°C.

5.9 kg of UNILINK 4200 was slowly added to this product under vigorous stirring. The reaction was exothermic and the temperature of the reaction mass was maintained at 70°C, the feeding rate of the amine being controlled.

The reaction mass was maintained at this temperature for 1.5 hours. The product (D) was subsequently discharged and analyzed, providing the following characteristics:
NCO % = 24.1;
Viscosity 25°C = 360 mPa.sec;
Aspect: yellow liquid;
Crystallization point = 10°C.

### EXAMPLE 5

17.52 kg of Tedimon 306, 17.52 kg of Tedimon 307 and 10 kg of Tedimon 31 preheated to 50°C, was charged into a reactor filled with nitrogen, equipped with stirring and a cooling system.

5.9 kg of UNILINK 4200 was slowly added to this product under vigorous stirring. The reaction was exothermic and the temperature of the reaction mass was maintained at 70°C, the feeding rate of the amine being controlled.

The reaction mass was maintained at this temperature for 1.5 hours. The product (E) was subsequently discharged and analyzed, providing the following characteristics:
NCO % = 26.7;
Viscosity 25°C = 250 mPa.sec;
Aspect: brown liquid;
Crystallization point = 5°C.

The compositions of Examples 1-5 were used for the preparation of flexible expanded polyurethane materials combined with the polyol component indicated in the following table. The same table also specifies the physico-mechanical characteristics of the foams and the results of the flame-reaction tests.

**TABLE**

| Composition | a | b | c | d | e |
|---|---|---|---|---|---|
| POLYOL PM 6000 | 100 | 100 | 100 | 100 | 100 |
| Chain extender | 2 | 2 | 1 | 1 | 1 |
| Amine catalyst | 0.23 | 0.23 | 0.25 | 0.25 | 0.25 |
| Stabilizer | 0.4 | 0.4 | 0.4 | 0.4 | 0.4 |
| Total water | 2.3 | 2.3 | 2.3 | 2.3 | 2.3 |
| Organometallic cat. | 0.1 | 0.1 | 0.12 | 0.12 | 0.12 |
| TDI 80/20 | 33.1 | | | | |
| Product C | | 42.3 | | | |
| POLYMERIC MDI | | | 36.2 | | |
| Prod.A/Prod.B (50/50) | | | | 40.0 | |
| Product D | | | | | 38.9 |
| Density kg/m³ | 43.1 | 46.5 | 60.9 | 55.4 | 54.7 |
| Comp.strength 40%,kPa | 2.4 | 2.6 | 5.4 | 4.1 | 2.1 |
| Elastic failure 50% | 3.8 | 5.1 | 7.5 | 12.9 | 10.8 |
| Elastic yield, % | 61 | 58 | 43 | 49 | 53 |
| Tensile strength, kPa | 89 | 90 | 43 | 74 | 80 |
| Ultimate elongation, % | 165 | 123 | 61 | 100 | 115 |
| MVSS 0302 Test | fail | pass | pass | pass | pass |
| California 117 | fail | pass | pass | pass | pass |
| CSE RF4/83, class. | fail | 3.1M | fail | 1.IM | 1.IM |

The following definitions are indicated in the table:
PM 6000 POLYOL: TERCAROL 427 of ENICHEM S.p.A.
POLYMERIC MDI: TEDIMON 31 of ENICHEM S.pA.
TDI 80/20: TEDIMON 80 of ENICHEM S.p.A.
Chain extender: Diethanolamine
Amine catalyst: DABC 33LV of AIR PRODUCTS
Organometallic catalyst: DABCO T12 of AIR PRODUCTS
Stabilizer: TEGOSTAB B 8681 of GOLDSCHMIDT

## Claims

1. A process for the preparation of a flexible expanded polyurethane material endowed with flame resistance, which comprises reacting:
a) an isocyanic component obtainable from the reaction of at least one organic isocyanate which is at least bifunctional, with a secondary amine having the general formula (I): 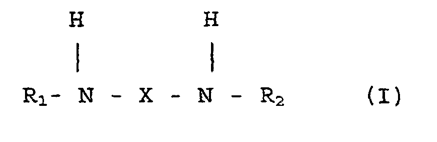
wherein R1 and R₂ independently are the same or different and represent a C₁-C₈ (iso)alkyl radical and X represents a 4,4'-methylenediphenylene radical, and wherein the molar ratio between the NCO groups of the said isocyanate and functional amine groups in the amine of formula (I) is from 2 to 10; with
b) a polyol component which comprises at least one polyol with a hydroxyl functionality from 2 to 8 and a molecular weight from 50 to 2,000.

2. A process according to claim 1, wherein in the secondary amine having general formula (I) at least one of R₁ and R₂ is an isobutyl radical.

3. A process according to claim 2 in which both R₁ and R₂ are isobutyl radicals.

4. The process according to any one of the preceding claims wherein the isocyanic component is selected from those with a low or medium molecular weight having general formula (II):
OCN - R - NCO (II)
wherein R represents a C₁-C₁₂ (iso) alkyl, a C₅-C₁₅ cycloalkyl or a C₆-C₁₈ aromatic radical which is optionally substituted with a C₁-C₄ alkyl radical or from polyisocyanates with a high molecular weight having general formula (III): 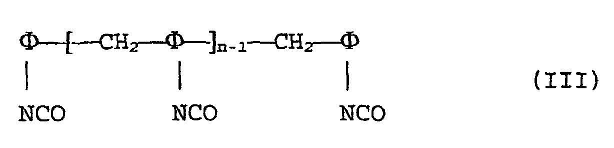
wherein Φ represents a phenyl group and n an integer greater than or equal to 1.

5. A process according to claim 4 wherein the polyisocyanate is a high molecular weight polyisocyanate obtained from the phosgenation of anilineformaldehyde condensates.

6. A process according to claim 4 wherein the polyisocyanate is a low or medium molecular weight polyisocyanate selected from hexamethylene diisocyanate, meta phenylene diisocyanate, para-phenylene diisocyanate, 2,4-toluenediisocyanate (TDI), 2,6-toluenediisocyanate, 4,4'-diphenylmethane-diisocyanate (MDI), 2,4' diphenylmethane-diisocyanate, 4,4'-dicyclohexylmethane diisocyanate, and 1-isocyanate-3-isocyanatemethyl-3,3,5-trimethylcyclohexane.

7. A process according to any one of claims 1 to 6, wherein the polyol is selected from polyol polyethers, polyol polyethers containing ester groups, polyol polyethers containing amine groups and polyol polyesters.

8. A process according to any one of the preceding claims wherein the polyol is selected from polyol polyethers obtained by the condensation of a C₂₋C₆ olefinic oxide with a compound having at least two active hydrogen atoms.

9. A process according to claim 8 wherein the olefinic oxide is selected from ethylene oxide and propylene oxide.

10. A process according to any one of claims 8 and 9 wherein the compound having at least two active hydrogen atoms is selected from a glycol, a triol, a tetrol, a diamine, an aromatic amine, an alkanol amine and a polyfunctional hydroxy alkane.

11. A process according to any one of the preceding claims wherein the polyurethane material is expanded by means of an expanding agent.

12. A process according to claim 11 wherein the expanding agent comprises water and optionally a hydrofluoroalkane, carbon dioxide, a hydrocarbon and mixtures thereof.

13. Use of an isocyanic component obtainable from the reaction of at least one organic isocyanate which is at least bifunctional, with a secondary amine having the general formula (I): 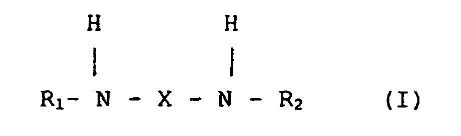
wherein R1 and R₂ independently are the same or different and represent a C₁₋C₈ (iso)alkyl radical and X represents a 4,4'-methylenediphenylene radical, and wherein the molar ratio between the NCO groups of the said isocyanate and functional amine groups in the amine of formula (I) is from 2 to 10 in the preparation of a flexible expanded polyurethane material endowed with flame-resistance.

14. Use of an isocyanic component according to claim 13, wherein the isocyanic component has an isocyanic functionality from 15 to 40%.

15. Use of an isocyanic component according to any one of claims 13 and 14, wherein the isocyanic component has a viscosity at 25°C from 40 to 10000 mPa.sec.

16. An expanded flexible polyurethane material having endowed flame resistance which is obtainable by a process according to any one of claims 1 to 12.

17. An expanded flexible polyurethane material according to claim 16 which passes the CSE RF4 test up to the classification 1.IM and which are substantially free of flame retardant of the halogenated or phospho-halogenated type

18. An article comprising an expanded flexible polyurethane material according to any one of claims 16 and 17.

## Revendications

1. Procédé de préparation d'un matériau souple en polyuréthane expansé doté d'une résistance à la flamme, comprenant la réaction :
a) d'un composant isocyanique pouvant être obtenu par la réaction d'au moins un isocyanate organique au moins bifonctionnel, avec une amine secondaire de formule générale (I) : 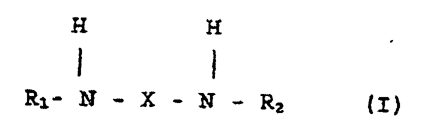
dans laquelle R₁ et R₂, indépendamment l'un de l'autre, sont identiques ou différents et représentent un radical (iso)alkyle en C₁-C₈, et X représente un radical 4,4'-méthylènediphénylène, et où le rapport molaire entre les groupes NCO dudit isocyanate et les groupes fonctionnels amine dans l'amine de formule (I) est de 2 à 10 ;
avec
b) un composant de type polyol comprenant au moins un polyol doté d'une fonctionnalité hydroxyle de 2 à 8 et dont la masse moléculaire est de 50 à 2 000.

2. Procédé selon la revendication 1, dans lequel dans l'amine secondaire de formule générale (I), au moins un élément parmi R₁ et R₂ représente un radical isobutyle.

3. Procédé selon la revendication 2, dans lequel à la fois R₁ et R₂ représentent des radicaux isobutyle.

4. Procédé selon l'une quelconque des revendications précédentes, dans lequel le composant isocyanique est choisi parmi ceux de masse moléculaire faible ou moyenne, de formule générale (II) :
OCN-R-NCO (II)
dans laquelle R représente un groupe (iso) alkyle en C₁-C₁₂, un groupe cycloalkyle en C₅-C₁₅ ou un radical aromatique en C₆-C₁₈, éventuellement substitué par un radical alkyle en C₁-C₄ ou parmi des polyisocyanates de masse moléculaire élevée, de formule générale (III) : 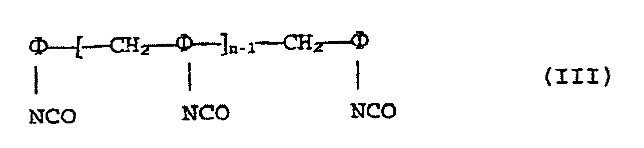
dans laquelle Φ représente un groupe phényle et n un nombre entier supérieur ou égal à 1.

5. Procédé selon la revendication 4, dans lequel le polyisocyanate est un polyisocyanate de masse moléculaire élevée, obtenu par phosgénation de condensats aniline-formaldéhyde.

6. Procédé selon la revendication 4, dans lequel le polyisocyanate est un polyisocyanate de masse moléculaire faible ou moyenne choisi parmi le diisocyanate d'hexaméthylène, le diisocyanate de métaphénylène, le diisocyanate de paraphénylène, le diisocyanate de 2,4-toluène (TDI), le diisocyanate de 2,6-toluène, le diisocyanate de 4,4'-diphénylméthane (MDI), le diisocyanate de 2,4'-diphénylméthane, le diisocyanate de 4,4'-dicyclohexylméthane et le 1-isocyanate-3-isocyanateméthyl-3,3,5-triméthylcyclohexane.

7. Procédé selon l'une quelconque des revendications 1 à 6, dans lequel le polyol est choisi parmi les polyétherpolyols, les polyétherpolyols comportant des groupes ester, les polyétherpolyols comportant des groupes amines et les polyesterpolyols.

8. Procédé selon l'une quelconque des revendications précédentes, dans lequel le polyol est choisi parmi les polyétherpolyols obtenus par condensation d'un oxyde oléfinique en C₂-C₆ avec un composé comportant au moins deux atomes d'hydrogène actif.

9. Procédé selon la revendication 8, dans lequel l'oxyde oléfinique est choisi parmi l'oxyde d'éthylène et l'oxyde de propylène.

10. Procédé selon l'une quelconque des revendications 8 ou 9, dans lequel le composé comportant au moins deux atomes d'hydrogène actif est choisi parmi un glycol, un triol, un tétrol, une diamine, une amine aromatique, une alcanolamine et un hydroxyalcane polyfonctionnel.

11. Procédé selon l'une quelconque des revendications précédentes, dans lequel le matériau en polyuréthane est expansé au moyen d'un agent expanseur.

12. Procédé selon la revendication 11, dans lequel l'agent expanseur comprend de l'eau et, éventuellement, un hydrofluoroalcane, du dioxyde de carbone, un hydrocarbure et des mélanges de ceux-ci.

13. Utilisation d'un composant isocyanique pouvant être obtenu par la réaction d'au moins un isocyanate organique au moins bifonctionnel, avec une amine secondaire de formule générale (I) : 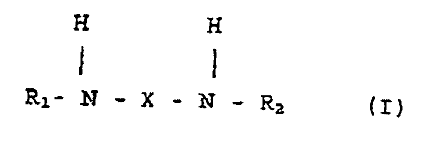
dans laquelle R₁ et R₂, indépendamment l'un de l'autre, sont identiques ou différents et représentent un radical (iso)alkyle en C₁-C₈, et X représente un radical 4,4'-méthylènediphénylène, et où le rapport molaire entre les groupes NCO dudit isocyanate et les groupes fonctionnels amine dans l'amine de formule (I) est de 2 à 10, dans la préparation d'un matériau souple en polyuréthane expansé doté d'une résistance à la flamme.

14. Utilisation d'un composant isocyanique selon la revendication 13, dans laquelle le composant isocyanique présente une fonctionnalité isocyanique de 15 à 40 %.

15. Utilisation d'un composant isocyanique selon l'une quelconque des revendications 13 ou 14, dans laquelle le composant isocyanique présente une viscosité à 25 °C de 40 à 10 000 mPa.s.

16. Matériau souple en polyuréthane expansé doté d'une résistance à la flamme pouvant être obtenu par un procédé selon l'une quelconque des revendications 1 à 12.

17. Matériau souple en polyuréthane expansé selon la revendication 16, classé 1.IM selon la norme CSE RF4, essentiellement dépourvu de toute préparation ignifuge de type halogéné ou phospho-halogéné.

18. Article comportant un matériau souple en polyuréthane expansé selon l'une quelconque des revendications 16 ou 17.

## Patentansprüche

1. Verfahren zur Herstellung eines flexiblen, geschäumten Polyurethanmaterials mit Flammbeständigkeit, umfassend das Reagieren
a) einer Isocyankomponente, erhältlich durch die Reaktion wenigstens eines organischen Isocyanats, das wenigstens bifunktionell ist, mit einem sekundären Amin der allgemeinen Formel (I): 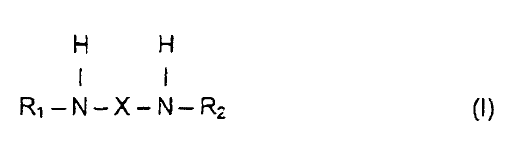
wobei R₁ und R₂ unabhängig voneinander gleich oder unterschiedlich sind und einen C₁-C₈-(iso)alkylrest darstellen, und X einen 4,4'-Methylendiphenylenrest darstellt, und wobei das Molverhältnis zwischen den NCO-Gruppen des Isocyanats und den funktionellen Amingruppen in dem Amin der Formel (I) 2 bis 10 beträgt; mit
b) einer Polyolkomponente, die wenigstens ein Polyol mit einer Hydroxylfunktionalität von 2 bis 8 und einem Molekulargewicht von 50 bis 2.000 umfasst.

2. Verfahren nach Anspruch 1, wobei wenigstens einer der R₁ und R₂ in dem sekundären Amin der allgemeinen Formel (I) ein Isobutylrest ist.

3. Verfahren nach Anspruch 2, wobei sowohl R₁ als auch R₂ Isobutylreste sind.

4. Verfahren nach einem der vorhergehenden Ansprüche, wobei die Isocyankomponente ausgewählt ist aus denjenigen Isocyankomponenten der allgemeinen Formel (II) mit einem niedrigen oder mittleren Molekulargewicht
OCN - R - NCO (II)
wobei R einen C₁-C₁₂-(Iso)alkyl-, einen C₅-C₁₅-Cycloalkyl- oder einen aromatischen C₆-C₁₈-Rest darstellt, der gegebenenfalls mit einem C₁-C₄-Alkylrest substituiert ist,
oder aus Polyisocyanaten der allgemeinen Formel (III) mit einem hohen Molekulargewicht 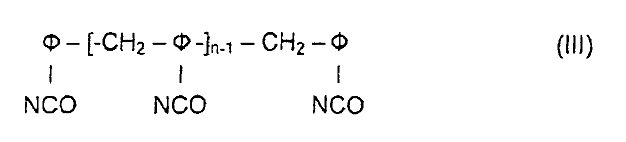
wobei Φ eine Phenylgruppe darstellt und n eine ganze Zahl größer als oder gleich 1 ist.

5. Verfahren nach Anspruch 4, wobei das Polyisocyanat ein hochmolekulares Polyisocyanat, erhältlich durch die Phosgenierung von Anilin-Formaldehyd-Kondensationsprodukten, ist.

6. Verfahren nach Anspruch 4, wobei das Polyisocyanat ein nieder- oder mittelmolekulares Polyisocyanat, ausgewählt aus Hexamethylendiisocyanat, meta-Phenylendiisocyanat, para-Phenylendüsocyanat, 2,4-Toluoldiisocyanat (TDI), 2,6-Toluoldiisocyanat, 4,4'-Diphenylmethandiisocyanat (MDI), 2,4'-Diphenylmethandiisocyanat, 4,4'-Dicyclohexylmethandiisocyanat und 1-Isocyanat-3-isocyanatmethyl-3,3,5-trimethylcyclohexan, ist.

7. Verfahren nach einem der Ansprüche 1 bis 6, wobei das Polyol ausgewählt ist aus Polyolpolyethern, Estergruppen-enthaltenden Polyolpolyethern, Amingruppen-enthaltenden Polyolpolyethern und Polyolpolyestern.

8. Verfahren nach einem der vorhergehenden Ansprüche, wobei das Polyol ausgewählt ist aus Polyolpolyethern, erhältlich durch die Kondensation eines olefinischen C₂-C₆-Oxids mit einer Verbindung mit wenigstens zwei aktiven Wasserstoffatomen.

9. Verfahren nach Anspruch 8, wobei das olefinische Oxid ausgewählt ist aus Ethylenoxid und Propylenoxid.

10. Verfahren nach einem der Ansprüche 8 und 9, wobei die Verbindung mit wenigstens zwei aktiven Wasserstoffatomen ausgewählt ist aus einem Glykol, einem Triol, einem Tetrol, einem Diamin, einem aromatischen Amin, einem Alkanolamin und einem polyfunktionellen Hydroxyalkan.

11. Verfahren nach einem der vorhergehenden Ansprüche, wobei das Polyurethanmaterial mit Hilfe eines Schaummittels geschäumt wird.

12. Verfahren nach Anspruch 11, wobei das Schaummittel Wasser und gegebenenfalls ein Hydrotluoralkan, Kohlendioxid, einen Kohlenwasserstoff und Mischungen davon umfasst.

13. Verwendung einer lsocyankomponente, erhältlich durch die Reaktion wenigstens eines organischen lsocyanats, das wenigstens bifunktionell ist, mit einem sekundären Amin der allgemeinen Formel (I): 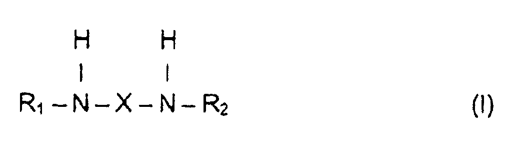
wobei R₁ und R₂ unabhängig voneinander gleich oder unterschiedlich sind und einen C₁-C₈-(lso)alkylrest darstellen, und X einen 4,4'-Methylendiphenylenrest darstellt, und wobei das Molverhältnis zwischen den NCO-Gruppen des Isocyanats und den funktionellen Amingruppen in dem Amin der Formel (I) 2 bis 10 beträgt, zur Herstellung eines flexiblen, geschäumten Polyurethanmaterials mit Flammbeständigkeit.

14. Verwendung einer Isocyankomponente nach Anspruch 13, wobei die Isocyankomponente eine Isocyanfunktionalität von 15 bis 40 % aufweist.

15. Verwendung einer Isocyankomponente nach einem der Ansprüche 13 und 14, wobei die Isocyankomponente bei 25 °C eine Viskosität von 40 bis 10.000 mPa.s aufweist.

16. Geschäumtes, flexibles Polyurethanmaterial mit Flammbeständigkeit, erhältlich durch ein Verfahren nach einem der Ansprüche 1 bis 12.

17. Geschäumtes, flexibles Polyurethanmaterial nach Anspruch 16, welches den CSE RF4 Test bis zu der Klassifikation 1.IM besteht und im wesentlichen frei von einem Flammschutzmittel der halogenierten oder phospho-halogenierten Art ist.

18. Artikel, umfassend ein geschäumtes, flexibles Polyurethanmaterial nach einem der Ansprüche 16 und 17.
